(19) **Europäisches Patentamt / European Patent Office / Office européen des brevets**

(11) **EP 3 632 455 A1**

(12) **EUROPEAN PATENT APPLICATION**
published in accordance with Art. 153(4) EPC

(43) Date of publication:
**08.04.2020 Bulletin 2020/15**

(21) Application number: **18810092.9**

(22) Date of filing: **30.05.2018**

(51) Int Cl.:
*A61K 36/185* (2006.01)  *A23L 33/105* (2016.01)
*A61K 36/06* (2006.01)  *A61P 1/04* (2006.01)
*A61P 1/12* (2006.01)  *A61P 43/00* (2006.01)

(86) International application number:
**PCT/JP2018/020866**

(87) International publication number:
**WO 2018/221627 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2017 JP 2017108131**

(71) Applicant: **Amino Up Co., Ltd.**
**Sapporo-shi**
**Hokkaido 004-0839 (JP)**

(72) Inventors:
• **HIRAYAMA Yosuke**
**Sapporo-shi**
**Hokkaido 004-0839 (JP)**

• **GOTO Kazunori**
**Sapporo-shi**
**Hokkaido 004-0839 (JP)**
• **TAKANO Sho**
**Sapporo-shi**
**Hokkaido 004-0839 (JP)**
• **TAKAHASHI Nodoka**
**Sapporo-shi**
**Hokkaido 004-0839 (JP)**

(74) Representative: **v. Bezold & Partner Patentanwälte - PartG mbB**
**Akademiestraße 7**
**80799 München (DE)**

(54) **PURSLANE PROCESSED MATERIAL, METHOD FOR PRODUCING PURSLANE PROCESSED MATERIAL, SUPPLEMENT, MEDICINE, INTESTINAL MUCOSA PROTECTIVE AGENT, AND INTESTINAL REGULATOR**

(57) The present disclosure provides a treated product of purslane characterized by being obtained by treating purslane with hot water to obtain a hot water extract, and adding yeast to the hot water extract and culturing the resultant.

## FIG.2A

EP 3 632 455 A1

**(Cont. next page)**

# FIG.2B

SEQUENCE LISTING

<110> AMINO UP CHEMICAL CO., LTD.

<120> Common Purslane extract, method for making Common Purslane
extract, supplement, medicine, intestinal mucosa protective agent
and drug for controlling intestinal function

<130> C001-PCT

<150> JP2017-108131
<151> 2017-05-31

<160> 10

<170> PatentIn version 3.5

<210> 1
<211> 19
<212> DNA
<213> Artificial Sequence

<220>
<223> MUC2 forward primer

<400> 1
cacctgtgcc ctggaaggc                                                    19

<210> 2
<211> 20
<212> DNA
<213> Artificial Sequence

<220>
<223> MUC2 reverse primer

<400> 2
cggtcacgtg gggcaggttc                                                   20

<210> 3
<211> 21
<212> DNA
<213> Artificial Sequence

<220>
<223> GAPDH forward primer

<400> 3
gaaggtcgga gtcaacggat t                                                 21

<210> 4
<211> 20
<212> DNA
<213> Artificial Sequence

<220>
<223> GAPDH reverse primer

<400> 4
cgctcctgga agatggtgat                                                   20

```
<210>   5
<211>   25
<212>   DNA
<213>   Artificial Sequence

<220>
<223>   Bact934F

<400>   5
ggarcatgtg gtttaattcg atgat                                              25


<210>   6
<211>   20
<212>   DNA
<213>   Artificial Sequence

<220>
<223>   Bact1060R

<400>   6
agctgacgac aaccatgcag                                                    20


<210>   7
<211>   25
<212>   DNA
<213>   Artificial Sequence

<220>
<223>   Firm934F

<400>   7
ggagyatgtg gtttaattcg aagca                                              25


<210>   8
<211>   20
<212>   DNA
<213>   Artificial Sequence

<220>
<223>   Firm1060R

<400>   8
agctgacgac aaccatgcac                                                    20


<210>   9
<211>   21
<212>   DNA
<213>   Artificial Sequence

<220>
<223>   All bacteria foward

<400>   9
actcctacgg gaggcagcag t                                                  21


<210>   10
```

```
<211>  23
<212>  DNA
<213>  Artificial Sequence

<220>
<223>  All bacteria reverse

<400>  10
agtattaccg cggctgctgg cac                                                  23
```

**Description**

Technical Field

The present disclosure relates to a treated product of purslane, a method of producing a treated product of purslane, a supplement, a pharmaceutical, an agent for protecting intestinal mucosa and an intestinal regulator.

Background Art

The surface of the intestinal tract is covered with protective mucosa so as to protect the intestinal tract from external stimulation and bacterial infection. The main components of the protective mucosa contributing to the physical barrier of the intestinal tract are mucins. There are 20 kinds of mucin genes, and these genes are categorized in two types, namely, secretory type and membrane type mucins. The mucin mainly expressed in the intestinal tract is MUC2, which is a secretory type mucin.

Further, the presence of IgA in the protective mucus, which functions as a barrier for the intestinal tract, is also drawing an attention. IgA immunologically prevents intestinal bacteria and toxins produced by intestinal bacteria from entering the living body.

Purslane (*Portulaca oleracea. L*) is a perennial plant belonging to the genus *Portulaca*, of the family Portulacaceae. Purslane has long been used as a medicinal herb effective for various kinds of diseases, such as injuries and sickness, beriberi, constipation, dental pain, and the like.

Non Patent Literature 1 discloses that a polysaccharide derived from purslane potentiated mucin secretion in enteritis model rats.

Non Patent Literature 2 discloses the administration of a purslane extract to humans for the purpose of preventing muscle pain due to exercise, and the fact that the administration of the purslane extract resulted in a decrease in the amount of serum IgA.

Citation List

Non Patent Literature

Non Patent Literature 1: Chinese Journal of Integrated Traditional and Western Medicine on Digestion, 2009, vol.17, no.2, p. 96 to 99, Effect of portulaca oleracea polysaccharide on treating ulcerative colitis induced by TNBs in rats, ZHANG Tao, WANG Xiao-ping, CHCN Jian-yong, PAN Feng

Non Patent Literature 2: Isokinetics and Exercise Science 2011, vol. 19, p. 199 to 206, Preventive effects of purslane extract on delayed onset muscle soreness induced by one session bench-stepping exercise

Summary of Invention

Technical Problem

However, a purslane extract or a treated product of purslane having an excellent effect of protecting intestinal mucosa has not been reported, so far.

The present inventors have found out that a treated product of purslane obtained by a predetermined production method using yeast has an excellent effect of protecting intestinal mucosa, thereby completing the present disclosure. An objective of the present disclosure is to provide: a treated product of purslane having an excellent effect of protecting intestinal mucosa; a supplement, a pharmaceutical, an agent for protecting intestinal mucosa and an intestinal regulator containing the same; as well as a method of producing the treated product of purslane.

Solution to Problem

In order to achieve the above described objective, a treated product of purslane according to Aspect 1 of the present disclosure is characterized by being obtained by treating purslane with hot water to obtain a hot water extract, and adding yeast to the hot water extract and culturing the resultant.

For example, at least one of pectinase or cellulase is further added to the hot water extract.

A method of producing a treated product of purslane, according to Aspect 2 of the present disclosure, includes:

a hot water treatment step of treating purslane with hot water; and
a culturing step of adding yeast, and culturing the resultant.

For example, at least one of pectinase or cellulase is further added, in the culturing step.
A supplement according to Aspect 3 of the present disclosure includes, as an effective ingredient, a treated product of purslane obtained by treating purslane with hot water to obtain a hot water extract, and adding yeast to the hot water extract and culturing the resultant.
For example, at least one of pectinase or cellulase is further added to the hot water extract.
A pharmaceutical according to Aspect 4 of the present disclosure includes, as an effective ingredient, a treated product of purslane obtained by treating purslane with hot water to obtain a hot water extract, and adding yeast to the hot water extract and culturing the resultant.
For example, at least one of pectinase or cellulase is further added to the hot water extract.
An agent for protecting intestinal mucosa according to Aspect 5 of the present disclosure includes, as an effective ingredient, a treated product of purslane obtained by treating purslane with hot water to obtain a hot water extract, and adding yeast to the hot water extract and culturing the resultant.
For example, at least one of pectinase or cellulase is further added to the hot water extract.
An intestinal regulator according to Aspect 6 of the present disclosure includes, as an effective ingredient, a treated product of purslane obtained by treating purslane with hot water to obtain a hot water extract, and adding yeast to the hot water extract and culturing the resultant.
For example, at least one of pectinase or cellulase is further added to the hot water extract.

Advantageous Effects of Invention

According to the present disclosure, it is possible to provide: a treated product of purslane having an excellent effect of protecting intestinal mucosa; a supplement, a pharmaceutical, an agent for protecting intestinal mucosa and an intestinal regulator containing the same; as well as a method of producing the treated product of purslane.

Brief Description of Drawings

FIG. 1 is a table showing MUC2 gene expression in the DLD-1 by each sample treatment;
FIG. 2(a) is a graph showing the results of analyzing MUC2 gene expression depending on the concentration of the treated product of purslane + yeast; and FIG. 2(b) is a graph showing the results of measuring the expression levels of MUC2 gene in the DLD-1, induced by a hot water extract of purslane alone, the treated product of purslane + yeast, and a hot water extract of yeast alone;
FIG. 3 is a graph showing the results of measuring the expression levels of MUC2 gene in the DLD-1, induced by a sample of the treated product of purslane + yeast which had not been treated with pectinase, and a sample of the treated product of purslane + yeast which had been treated with at least one of pectinase or cellulase;
FIG. 4 is a graph showing the results of examining the relationship between the amount of yeast added to purslane in the production process of the treated product of purslane + yeast and the expression level of MUC2 gene in the DLD-1;
FIG. 5 is a graph showing the results of examining the relationship between culture time after adding yeast to purslane in the production process of the treated product of purslane + yeast and the expression level of MUC2 gene in the DLD-1;
FIG. 6(a) is a graph showing the results of measuring the amount of fecal mucin in an animal test, 14 days after the start of the test, and FIG. 6(b) is a graph showing the results of measuring the amount of fecal sIgA, 14 days after the start of the test;
FIG. 7(a) and FIG. 7(b) include graphs showing the results of a simple microbiota analysis using a quantitative PCR in the animal test, and FIG. 7(a) shows the analysis results of bacteria belonging to the phylum Bacteroidetes, and FIG. 7(b) shows the analysis results of bacteria belonging to the phylum Firmicutes;
FIG. 8(a) is a graph showing the results of measuring the amount of fecal mucin, in an in-house monitor test (three subjects), three weeks after the start of the test; and FIG. 8(b) is a graph showing the results of measuring the amount of fecal sIgA three weeks after the start of the test;
FIG. 9(a) is a diagram explaining a visual analog scale (VAS) scale, and FIG. 9(b) is a graph showing the results of carrying out a VAS questionnaire;
FIG. 10 is a table showing the results of the questionnaire regarding defecation status;
FIG. 11(a) is a diagram showing the results of a comprehensive analysis of intestinal microbiota (genus level) in the in-house monitor test (three subjects), and FIG. 11(b) is a graph showing the results of a principal component analysis of intestinal microbiota, in the test;

FIG. 12(a) is a table showing a diversity index of intestinal bacteria, in the in-house monitor test (three subjects), and FIG 12(b) is a table showing Firmicutes/Bacteroidetes (FB) ratios in the test;

FIG. 13(a) is a table showing the answered results regarding the sense of change, in an in-house monitor test (37 subjects), FIG. 13(b) is a table showing the answered results regarding dietary habit, and FIG. 13(c) is a table showing the relationship between the time of ingestion and the sense of change;

FIG. 14 is a table showing improved items in the in-house monitor test (37 subjects);

FIG. 15 is a graph showing the results of measuring the amount of fecal mucin;

FIG. 16 is a graph showing the results of patient assessment of constipation quality of life (PAC-QOL) score; and

FIG. 17(a) is a graph showing the results of psychological discomfort score in PAC-QOL, and FIG. 17(b) is a graph showing the results of anxiety score in PAC-QOL.

Description of Embodiments

First, the method of producing a treated product of purslane according to the present embodiment will be described in detail.

The method of producing the treated product of purslane according to the present embodiment includes: a hot water treatment step of treating purslane with hot water; and a culturing step of adding yeast, and culturing the resultant. In the present specification, an extract derived from purslane, obtained through the hot water treatment step, is also referred to as "hot water extract" or "hot water extract of purslane". Further, in the present specification, a treated product derived from purslane, obtained through the culturing step, is also referred to as "treated product of purslane" or "treated product of purslane + yeast".

Purslane (*Portulaca oleracea. L*) is a perennial plant belonging to the genus *Portulaca*, of the family *Portulacaceae*, which is closely related to the family Cactaceae. Purslane comprises leaves, stems and seeds. The leaves and stems are moderately thick and contain a viscous substance, and are sometimes used as food and herbal medicine. Purslane grows widely around the world, from the tropics to the temperate zone, and is resistant to high temperature and dry environments. It is possible to use any part of purslane, but it is preferred to use the above ground part of purslane. Particularly preferred are stems and leaves. These parts can be used with or without drying. In the present embodiment, purslane which has been dried (dried purslane: for example, in a state having a water content of from 0 to 10% by weight) can be suitably used, and the dried purslane may further be crushed before use.

The amount of water to be used for the hot water treatment of purslane is from 1 to 30 times, preferably from 10 to 30 times the amount of purslane (such as dried purslane). In the hot water treatment, a saccharide may be added in order to increase the reaction velocity of yeast. The saccharide to be added is preferably a disaccharide such as sucrose (for example, sucrose is added in an amount of 15% by weight or less with respect to the amount of water). Further, purslane, water and a saccharide may be added in any order.

The hot water treatment step is carried out by adding the above described amount of water to purslane, and, for example, heating the purslane in hot water at a temperature of from 100 to 130°C for 15 to 30 minutes (for example, at 121°C for 20 minutes). It is preferred that sterilization or disinfection be achieved in the hot water treatment step. After the hot water treatment step, the resulting hot water extract is cooled to a temperature of 60°C or less, preferably to 25 to 40°C, and then yeast is added to the hot water extract. The hot water extract may be cooled by any method, and may be allowed to cool naturally, or cooled by a positive cooling method such as water cooling.

After adding yeast to the hot water extract obtained through the hot water treatment step, the resultant is cultured. The culture is carried out at a temperature of from 25 to 40°C for 16 hours or more. It is preferred that the culture be carried out by shaking culture or while stirring. The culture is carried out at a temperature of from 25 to 40°C, and preferably from 30 to 37°C. The culture is carried out for 16 hours or more. However, the culture time is preferably from 16 to 72 hours, and more preferably from 16 to 24 hours, from the viewpoint of production cost.

The yeast to be used is not particularly limited, as long as the yeast allows for achieving the effect of the present disclosure. However, an easily usable dry yeast may be used. For example, it is possible to use, as the dry yeast, Nissin Super Camelia dry yeast (manufactured by Oriental Yeast Co., Ltd.), or Saf-Instant Dry Yeast (manufactured by Lesaffre Yeast Corporation). The amount of yeast to be added is 7.5% by weight or more of the amount of purslane (such as dried purslane). From the viewpoint of production efficiency, the amount of yeast to be added is preferably from 65 to 260% by weight, and more preferably from 70 to 90% by weight of the amount of purslane.

Further, at least one of pectinase or cellulase may be added in the culturing step. Pectinase and cellulase are enzymes that break down cell walls of higher plants. The addition of at least one of pectinase or cellulase enables to efficiently treat purslane. Pectinase and cellulase to be used are not particularly limited, as long as at least one of pectinase or cellulase allow for achieving the effect of the present disclosure. For example, it is possible to use Pectinase PL Amano (manufactured by Amano Enzyme Inc.), and/or Cellulase A Amano 3 (manufactured by Amano Enzyme Inc.).

The amount of at least one of pectinase or cellulase to be added is, for example, from 5 to 20% by weight, and is preferably from 5 to 15% by weight of the amount of purslane (such as dried purslane) from the viewpoint of production cost. At least one of pectinase and cellulase may be added before or after, or at the same time as, adding yeast. However, in particular, it is preferred that the temperature of the hot water extract of purslane or the treated product of purslane when adding pectinase be 60°C or less; the temperature of the hot water extract of purslane or the treated product of purslane when adding cellulase be 70°C or less; and the temperature of the hot water extract of purslane when adding yeast be 60°C or less. For example, pectinase is added after cooling the hot water extract of purslane or the treated product of purslane to a temperature of from 45 to 50°C, and yeast is added after further cooling the resultant to 25 to 40°C.

The culture liquid obtained after the culturing step may be subjected to a heat treatment again (for example, at a temperature of from 100 to 130°C for 15 to 30 minutes). By carrying out a heat treatment after the culture, it is possible to sterilize the added yeast, and, in the case of adding at least one of pectinase or cellulase, to inactivate the at least one of pectinase or cellulase.

After the culturing step, the solid content may be removed. For example, after removing residues by a centrifuge, a decanter or the like, solid-liquid separation may be carried out by suction filtration, or using a clarifier or the like, so as to achieve clarification. Subsequently, for example, the resultant may be concentrated to 2 to 15 times, and an excipient may be added to the resulting concentrate, followed by sterilization (for example, at 121°C for 15 to 30 minutes). Examples of the excipient include dextrin, starch, maltose, and sorbitol. Thereafter, for example, a drying treatment such as spray drying or freeze drying may further be carried out, followed by a sieving treatment.

One embodiment of the method of producing the treated product of purslane is as follows:

1. adding water in an amount of from 10 to 30 times the amount of dried purslane, to dried purslane, and sucrose (in an amount of 15% by weight or less of the amount of water);
2. carrying out a heat treatment (at 100 to 130°C for 15 to 30 minutes);
3. allowing the resultant to cool to 45 to 50°C, followed by the addition of pectinase (product name: Pectinase PL Amano, manufactured by Amano Enzyme Inc.) in an amount of from 5 to 20% by weight of the amount of dried purslane;
4. allowing the resultant to cool to 25 to 40°C, followed by the addition of dry yeast (product name: Saf-Instant Dry Yeast Gold Label, manufactured by Lesaffre Yeast Corporation) in an amount of from 7.5 to 260% by weight of the amount of dried purslane;
5. carrying out the culture and reaction (at 25 to 40°C for 16 to 72 hours, by shaking culture);
6. carrying out a heat treatment (at 100 to 130°C for 15 to 30 minutes);
7. separating the solid content by a decanter, followed by clarification using a clarifier;
8. concentrating the resultant 2 to 15 times, followed by the addition of dextrin (product name: Pinedex, manufactured by Matsutani Chemical Industry Co., Ltd.);
9. performing sterilization (at 121°C for 15 to 30 minutes); and
10. spray drying the resultant, followed by sieving.

One example the method of producing the treated product of purslane is as follows:

1. adding water in an amount of 15 times the amount of dried purslane, to dried purslane, and sucrose (in an amount of 2% by weight of the amount of water);
2. carrying out a heat treatment (at 121°C for 20 minutes);
3. allowing the resultant to cool to 50°C, followed by the addition of pectinase (product name: Pectinase PL Amano, manufactured by Amano Enzyme Inc.) in an amount of 10% by weight of the amount of dried purslane;
4. allowing the resultant to cool to 37°C, followed by the addition of dry yeast (product name: Saf-Instant Dry Yeast Gold Label, manufactured by Lesaffre Yeast Corporation) in an amount of 80% by weight of the amount of dried purslane;
5. carrying out the culture and reaction (at 37°C for 20 hours, by shaking culture);
6. carrying out a heat treatment (at 121°C for 20 minutes);
7. separating the solid content by a decanter, followed by clarification using a clarifier;
8. concentrating the resultant 10 times, followed by the addition of dextrin (product name: Pinedex, manufactured by Matsutani Chemical Industry Co., Ltd.);
9. performing sterilization (at 121°C for 20 minutes); and
10. spray drying the resultant, followed by sieving.

Next, the treated product of purslane according to the present embodiment will be described.

The treated product of purslane according to the present embodiment is produced using purslane as a raw material and by the above described production method, and is obtained by treating purslane with hot water to obtain a hot water extract (hot water extract of purslane), and adding yeast to the hot water extract and culturing the resultant. In the present specification, the treated product of purslane is also referred to as "treated product of purslane + yeast". In cases where pectinase is added in the culturing step, the treated product of purslane is also referred to as "pectinase-treated product of purslane + yeast".

The treated product of purslane according to the present embodiment has the effect of increasing mucin gene expression, the effect of increasing the amount of IgA, and the effect of improving the intestinal microbiota, and thus can be used as a supplement, a pharmaceutical, an agent for protecting intestinal mucosa, or an intestinal regulator, as described below.

Next, the supplement, the pharmaceutical, the agent for protecting intestinal mucosa and the intestinal regulator according to the present embodiment will be described.

The supplement, the pharmaceutical, the agent for protecting intestinal mucosa and the intestinal regulator according to the present embodiment each includes, as an effective ingredient, the treated product of purslane according to the present embodiment.

The supplement and the pharmaceutical according to the present embodiment can be used for the purpose of: regulating intestinal function; facilitating defecation; improving the intestinal microbiota; increasing the amount of feces; increasing the amount of fecal mucin, increasing the amount of fecal IgA, increasing useful intestinal bacteria, improving the intestinal environment, stimulating intestinal immunity, and the like. Further, the supplement and the pharmaceutical according to the present embodiment are expected to improve intestinal dysfunction such as diarrhea and dyschezia; as well as to improve, alleviate, treat and prevent various kinds of diseases and abnormalities related to abnormalities in the intestinal tissue, such as: intestinal immunodeficiency; the generation of metabolites of harmful microorganisms and the generation of harmful enzymes in the intestine, life style related diseases; and allergies. In addition, the supplement and the pharmaceutical according to the present embodiment are expected to be effective for conditions (such as Crohn's disease) where mucin gene expression is decreased.

The agent for protecting intestinal mucosa according to the present embodiment has the function of protecting intestinal mucosa, due to excellent effects of the treated product of purslane, as the effective ingredient, to increase mucin gene expression, to increase the amount of IgA, and to improve the intestinal microbiota. This eventually provides the effects of: regulating intestinal function; facilitating defecation; improving the intestinal microbiota; increasing the amount of feces; increasing the amount of fecal mucin, increasing the amount of fecal IgA, increasing useful intestinal bacteria, improving the intestinal environment, stimulating intestinal immunity, and the like. Further, the agent for protecting intestinal mucosa according to the present embodiment is expected to improve intestinal dysfunction such as diarrhea and dyschezia; as well as to improve, alleviate, treat and prevent various kinds of diseases and abnormalities related to abnormalities in the intestinal tissue, such as: intestinal immunodeficiency; the generation of metabolites of harmful microorganisms and the generation of harmful enzymes in the intestine, life style related diseases; and allergies. In addition, the agent for protecting intestinal mucosa according to the present embodiment is expected to be effective for conditions (such as Crohn's disease) where mucin gene expression is decreased.

The intestinal regulator according to the present embodiment provides the effects of: regulating intestinal function; facilitating defecation; improving the intestinal microbiota; increasing the amount of feces; increasing the amount of fecal mucin, increasing the amount of fecal IgA, increasing useful intestinal bacteria, improving the intestinal environment, stimulating intestinal immunity, and the like. This is due to excellent effects of the treated product of purslane, as the effective ingredient, to increase mucin gene expression, to increase the amount of IgA, and to improve the intestinal microbiota. Further, the intestinal regulator according to the present embodiment is expected to improve intestinal dysfunction such as diarrhea and dyschezia; as well as to improve, alleviate, treat and prevent various kinds of diseases and abnormalities related to abnormalities in the intestinal tissue, such as: intestinal immunodeficiency; the generation of metabolites of harmful microorganisms and the generation of harmful enzymes in the intestine, life style related diseases; and allergies. In addition, the intestinal regulator according to the present embodiment is expected to be effective for conditions (such as Crohn's disease) where mucin gene expression is decreased.

The supplement, the pharmaceutical, the agent for protecting intestinal mucosa and the intestinal regulator according to the present embodiment can be processed into forms suitable for food and beverage, such as, for example, granules, particles, tablets, capsules, gels, creams, pastes, suspensions, aqueous solutions, emulsions, powders and the like, by ordinary methods. Further, the supplement, the pharmaceutical, the agent for protecting intestinal mucosa and the intestinal regulator can contain an excipient, a binder, a lubricant, a colorant, a disintegrating agent, a thickener, a preserving agent, a stabilizing agent, a pH regulator and/or the like, usually used therein. For the purpose of improving the taste, the supplement, the pharmaceutical, the agent for protecting intestinal mucosa and the intestinal regulator can further contain a saccharide, a sugar alcohol, a salt, a fat or oil, an amino acid, an organic acid, glycerin and/or the like, as long as the effect of the present disclosure is not impaired. In the case of the pharmaceutical, the pharmaceutical can be prepared into any dosage form by an ordinary method, and examples of the dosage form include tablets, granules, powders, capsules, syrups and injectable solutions. Further, the pharmaceutical can contain an excipient, a binder, a lubricant, a colorant, a disintegrating agent, a thickener, a preserving agent, a stabilizing agent, a pH regulator and/or the like, usually used therein. The mode of administration can be selected as appropriate, as long as the effect of the present disclosure can be achieved, and examples of the administration mode include oral administration, intravenous administration, intraperitoneal administration, intradermal administration and sublingual administration.

The supplement, the pharmaceutical, the agent for protecting intestinal mucosa or intestinal regulator according to the present embodiment can be administered, for example, from 50 mg to 2,000 mg/day, preferably from 100 mg to 1,000 mg/day, and more preferably from 300 to 500 mg/day, in terms of the solid content of the treated product of purslane. The dosage thereof can be selected as appropriate, depending on the purpose of administration, the dosage form, the age and body weight of each patient, and the like.

As described above, the treated product of purslane according to the present embodiment has excellent effects of increasing mucin gene expression, increasing the amount of IgA and improving the intestinal microbiota, and the supplement, the pharmaceutical, the agent for protecting intestinal mucosa and the intestinal regulator, each containing the same as an effective ingredient, are provided. Further, the method of producing the treated product of purslane according to the present embodiment enables to produce the treated product of purslane having excellent effects of increasing mucin gene expression, increasing the amount of IgA and improving the intestinal microbiota, easily and at a low cost.

EXAMPLES

The present disclosure will now be described specifically by way of Examples. It is noted, however, that the present disclosure is in no way limited to these Examples.

(Example 1)

In vitro screening was carried out to analyze the level of mucin gene expression, using various kinds of samples.

As the samples, about 50 kinds of food samples were evaluated. The method of evaluating the expression level of mRNA of the mucin gene (MUC2), using human colon adenocarcinoma cell line (DLD-1 cells), will be described below. DLD-1 cells (provided by Institute of Development, Aging and Cancer, Tohoku University) were suspended in an RPMI 1640 medium (product name: RPMI 1640 medium "Nissui" 2 powder, manufactured by Nissui Pharmaceutical Co., Ltd.) supplemented with a 10% Fetal Bovine Serum (FBS). The resulting suspension was transferred into wells of 24-well microplates (25,000 cells/well), and cultured overnight at 37°C, in the presence of 5% $CO_2$. The supernatant was removed and a fresh RPMI 1640 medium was added. Then the respective samples were added to the wells so as to achieve a final concentration of 100 $\mu$g/mL. To the non-treated control, the RPMI 1640 medium was added. After culturing the cells for four hours at 37°C, in the presence of 5% $CO_2$, the supernatant was removed from the wells, and the cells were collected to be used for mRNA detection. From the collected cells, total RNA was extracted using TRIzol reagent (manufactured by Life Technologies Corporation), and the measurement of the concentration was carried out using Nanodrop (manufactured by Thermo Fisher Scientific). Subsequently, cDNA was synthesized, using cDNA synthesis kit (product name: ReverTra Ace qPCR RT Master Mix with gDNA Remover, manufactured by TOYOBO Life Science Department). The reaction liquid obtained after reverse transcription was diluted with Nuclease-free water to a concentration of 3 ng/$\mu$L, to be used as a template for real-time PCR.

In the real-time PCR reaction, MUC2 forward primer (SEQ ID NO. 1), and MUC2 reverse primer (SEQ ID NO. 2) were used as primers. Glyceraldehyde-3-phosphate dehydrogenase (GAPDH) gene was used as an internal standard gene for correcting the expression of MUC2 gene, and GAPDH forward primer (SEQ ID No. 3) and GAPDH reverse primer (SEQ ID No. 4) were used as the primers for the gene. The real-time PCR reaction was carried out by a real-time PCR analysis system (product name: CFX Connect, manufactured by Bio-Rad Laboratories, Inc.), using a real-time reaction kit (product name: SsoAdvanced SYBR Green Supermix, manufactured by Bio-Rad Laboratories, Inc.). A total of 10 $\mu$L of the resulting PCR reaction liquid was incubated at 95°C for three minutes (initial denaturation). Thereafter, denaturation at 95°C for 10 seconds and annealing at 60°C for 30 seconds were repeated for 40 cycles.

    SEQ ID NO. 1: 5'-CACCTGTGCCCTGGAAGGC-3'
    SEQ ID NO. 2: 5'-CGGTCACGTGGGGCAGGTTC-3'
    SEQ ID NO. 3: 5'-GAAGGTCGGAGTCAACGGATT-3'
    SEQ ID NO. 4: 5'-CGCTCCTGGAAGATGGTGAT-3'

The expression ratio of MUC2 gene was calculated using Cq values obtained by a real-time PCR analysis system, CFX Connect, based on the following equations ($\Delta\Delta$Ct method):

    Cq value of control MUC2: A
    Cq value of control GAPDH: B

Cq value of sample MUC2: C
Cq value of sample GAPDH: D

$$\Delta Cq \,(\text{control}) = A - B$$

$$\Delta Cq \,(\text{sample}) = C - D$$

$$\Delta(\Delta Cq) = \Delta Cq \,(\text{sample}) - \Delta Cq \,(\text{control})$$

Expression ratio = $2^{-\Delta(\Delta Cq)}$

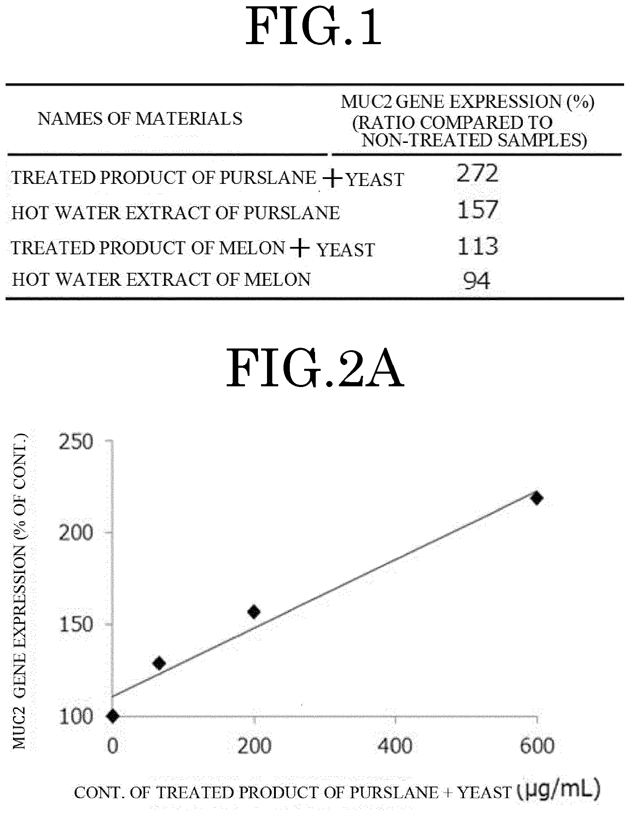

The "treated product of purslane + yeast" shown in FIGs. 1 to 3 was prepared as follows:

1. adding water in an amount of 15 times the amount of dried purslane, to dried purslane, and sucrose (in an amount of 2% by weight of the amount of water);
2. carrying out a heat treatment (at 121°C for 20 minutes);
3. allowing the resultant to cool to 37°C, followed by the addition of dry yeast (product name: Nissin Super Camelia dry yeast (manufactured by Oriental Yeast Co., Ltd.) in an amount of 80% by weight of the amount of dried purslane;
4. carrying out the culture and reaction (at 37°C for 20 hours, by shaking culture);
5. carrying out a heat treatment (at 121°C for 20 minutes);
6. separating the solid content by a decanter, followed by clarification using a clarifier;
7. concentrating the resultant 10 times, followed by the addition of dextrin (product name: Pinedex, manufactured by Matsutani Chemical Industry Co., Ltd.);
8. performing sterilization (at 121°C for 20 minutes); and
9. spray drying the resultant, followed by sieving.

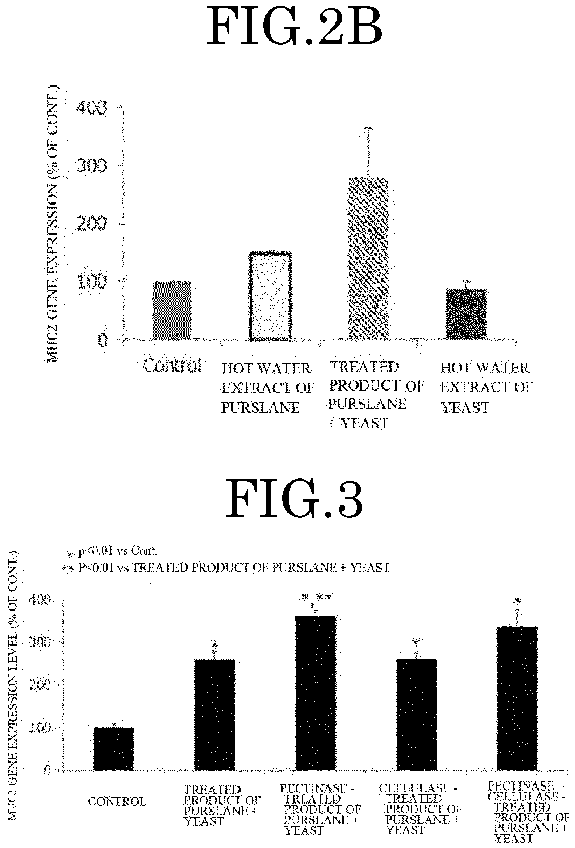

The "hot water extract of purslane" shown in FIGs. 1 and 2 was prepared by the above described method of preparing the "treated product of purslane + yeast", except that the addition of sucrose in the step "1.", as well as the steps "3.", "4." and "5.", were not carried out.

The results are shown in FIG. 1. An extremely high level of MUC2 gene expression was observed in the DLD-1 added the treated product of purslane + yeast, as compared to the hot water extract of purslane. It has also been observed that the expression level of MUC2 gene in the DLD-1 is increased in the treated product of melon + yeast, as compared to the hot water extract of melon. However, it can be seen from FIG 1 that an increase in the expression level of MUC2 gene due to the addition of yeast is an extremely large in purslane.

A concentration dependent test was carried out for the treated product of purslane + yeast, in which a marked increase in the expression level of MUC2 gene was observed. The expression level of MUC2 gene was measured in the same manner as described above, in samples each having a concentration of the treated product of purslane + yeast of 0, 66.6, 200 or 600 μg/mL. The results of the MUC2 gene expression level, when the sample having a concentration of the treated product of purslane + yeast of 0 μg/mL was taken as Control, are shown in FIG. 2(a). The treated product of purslane + yeast increased the expression level of MUC2 gene in a concentration dependent manner.

Further, the expression levels of MUC2 gene in the DLD-1 added the hot water extract of purslane, the treated product of purslane + yeast, and the hot water extract of yeast, were measured in the same manner as described above, and the measured expression levels were compared. The hot water extract of yeast was prepared by: adding water to dry yeast; carrying out a heat treatment at 121°C for 20 minutes; and then separating the solid content, followed by a drying treatment. The results of the MUC2 gene expression level in these DLD-1s, when a sample to which none of the above had been added was taken as Control, are shown in FIG. 2(b). It has been shown that the highest expression level of MUC2 gene is observed in the DLD-1 added the treated product of purslane + yeast, and that the expression level of MUC2 gene in the DLD-1 added the treated product of purslane + yeast is higher than the total value of the expression level of MUC2 gene in the DLD-1 added the hot water extract of purslane and the hot water extract of yeast. This revealed that the co-culture with yeast resulted in an increased activity.

Further, a sample of the treated product of purslane + yeast, which had not been treated with pectinase (prepared by the above described method of preparing the "treated product of purslane + yeast"), and enzyme-treated samples of the treated product of purslane + yeast (each prepared by the above described method of preparing the "treated product of purslane + yeast", except that: a step of allowing the resultant cool to 50°C, followed by the addition of at least one of pectinase (product name: Pectinase PL Amano, manufactured by Amano Enzyme Inc.) or cellulase (product name: Cellulase A Amano 3, manufactured by Amano Enzyme Inc.) in an amount of 10% by weight of the amount of dried purslane, was carried out between the step "2." and the step "3."; and that the culture and reaction were carried out in the step "3.") were prepared. The expression levels of MUC2 gene in DLD-1 treated with these samples were measured in the same manner as described above, and the measured expression levels were compared. The results of the MUC2 gene expression level, when a sample to which none of the above had been added was taken as Control, are shown in FIG. 3. It has been shown that an extremely high level of MUC2 gene expression is observed in the DLD-1 added the pectinase-treated product, as compared to the non-enzyme-treated product, revealing that the pectinase treatment resulted in a markedly increased activity. Further, the results revealed that the cellulase treatment as well as co-treatment with pectinase and cellulase also resulted in an increased activity, as compared to the case of carrying out no enzyme treatment.

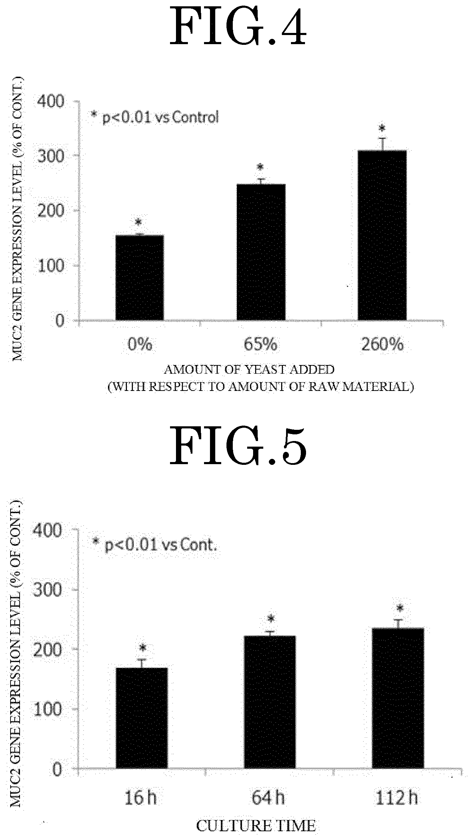

In addition, an examination was carried out on the amount of yeast to be added to purslane in the production process of the treated product of purslane + yeast. The expression level of MUC2 gene was measured in the same manner as described above, in samples to each of which dry yeast in an amount of 0%, 65% or 260% of the amount of dried purslane had been added, and the measured expression levels were compared. The results of the MUC2 gene expression level, when the sample to which the treated product of purslane + yeast had not been added was taken as Control, are shown in FIG. 4. As a result, it has been shown that the expression level of MUC2 gene increases in a manner dependent on the amount of yeast added.

An examination was also carried out on the culture time of the treated product of purslane + yeast. The expression level of MUC2 gene in the DLD-1 treated with samples cultured for 16 hours, 64 hours and 112 hours after adding dry yeast to purslane was measured in the same manner as described above, and the measured expression levels were compared. The results of the MUC2 gene expression level, when a sample to which the treated product of purslane + yeast had not been added was taken as Control, are shown in FIG. 5. As a result, it has been shown that the expression level of MUC2 gene increases in a manner dependent on the length of the culture time.

(Example 2)

Using a treated product of purslane + yeast prepared in the same manner as Example 1, an animal test was carried out as follows.

Dextran sulfate sodium (DSS) was administered to BALB/c mice (male, 6-week-old) to induce a moderate level of inflammation, to prepare model mice with reduced intestinal barrier function.

The mice were divided in the following groups:
˙ Control group: n = 10
˙ DSS-administered group: n = 10
˙ DSS + "treated product of purslane + yeast" (500 mg/kg) -administered group: n = 10

The test schedule is shown below. On days 0, 7 and 14 after the start of the test, feces were collected from mice in the respective groups.
˙ Control group: Fourteen days after the start of the test, the mice were sacrificed and dissected to collect the intestines.
˙ DSS-administration group: Seven days after the start of the test, the administration of 1.5% DSS was started. Fourteen days after the start of the test, the mice were sacrificed and dissected to collect the intestines.
˙ DSS + "treated product of purslane + yeast"-administered group: From the day of the start of the test, the administration of the "treated product of purslane + yeast" was started. Seven days after the start of the test, the administration of 1.5% DSS was started. Fourteen days after the start of the test, the mice were sacrificed and dissected to collect the intestines and feces.

The evaluation was carried out on the following points.
˙ Length of colon
After sacrificing the mice by bleeding under isoflurane anesthesia, the alimentary tract from the duodenum to the rectum was extracted from each mouse. The colon was cut out from the extracted alimentary tract, and the length thereof was measured by a scale.
˙ Amount of fecal mucin
The amount of fecal mucin was measured using a kit for measuring fecal mucin (product name: Fecal Mucin Assay Kit, manufactured by Cosmo Bio Co., Ltd.).

Amount of fecal sIgA

The amount of fecal sIgA was measured as an index for evaluating immunity. A quantity of 500 $\mu$L of saline

was added to the collected feces, and the feces were crushed with a spatula, followed by centrifugation (at 20,100 × g, 4°C, for 10 minutes). The resulting supernatant was used for an analysis using an IgA measurement kit (product name: Mouse IgA ELISA Ready-SET-Go!, manufactured by eBioScience).

. Simple microbiota analysis

The analysis was carried out by real-time PCR, using primers species-specific to the phyla Bacteroidetes and Firmicutes. DNA was extracted from the feces collected using a fecal DNA extraction kit (product name: PowerFecal DNA Isolation Kit, manufactured by MO BIO Laboratories, Inc.). A quantity of 50 pg/μL of the extracted DNA was diluted with Nuclease-free water, to be used as a template for PCR. The primers reported by Taira and others (Taira, 2015, J Clin Biochem Nutr, 56:149-54) were used for the PCR reaction. Bact934F (SEQ ID No. 5) and Bact1060R (SEQ ID No. 6) were used as phylum Bacteroidetes specific primers. Firm934F (SEQ ID No. 7) and Firm 1060R (SEQ ID No. 8) were used as phylum Firmicutes specific primers. Universal primers for the 16S rRNA region (all bacteria forward: SEQ ID No. 9, all bacteria reverse: SEQ ID No. 10) were used for measuring the total number of bacteria. The real-time PCR reaction was carried out using a real-time reaction kit (product name: SsoAdvanced SYBR Green Supermix, manufactured by Bio-Rad Laboratories, Inc.), by a real-time PCR analysis system (product name: CFX Connect, manufactured by Bio-Rad Laboratories, Inc.). A total of 10 μL of the PCR reaction liquid was incubated at 95°C for three minutes (initial denaturation). Subsequently, denaturation at 95°C for 5 seconds and annealing at 60°C for 15 seconds were repeated for 40 cycles.

SEQ ID No. 5: 5'-GGARCATGTGGTTTAATTCGATGAT-3'
SEQ ID No. 6: 5'-AGCTGACGACAACCATGCAG-3'
SEQ ID No. 7: 5'-GGAGYATGTGGTTTAATTCGAAGCA-3'
SEQ ID No. 8: 5'-AGCTGACGACAACCATGCAC-3'
SEQ ID No. 9: 5'-ACTCCTACGGGAGGCAGCAGT-3'
SEQ ID No. 10: 5'-AGTATTACCGCGGCTGCTGGCAC-3'

The expression ratio of MUC2 gene was calculated, using Cq values obtained by a real-time PCR analysis system, CFX Connect, based on the following equations (ΔΔCt method).
Cq value of phylum Bacteroidetes or Firmicutes: A
Cq value of total number of bacteria: B

$$\Delta Cq \text{ (total number of bacteria)} = B - B$$

$$\Delta Cq \text{ (phylum Bacteroidetes or Firmicutes)} = A - B$$

$$\Delta(\Delta Cq) = \Delta Cq \text{ (phylum Bacteroidetes or Firmicutes)} - \Delta Cq \text{ (total number of bacteria)}$$

Expression ratio = $2^{-\Delta(\Delta Cq)}$

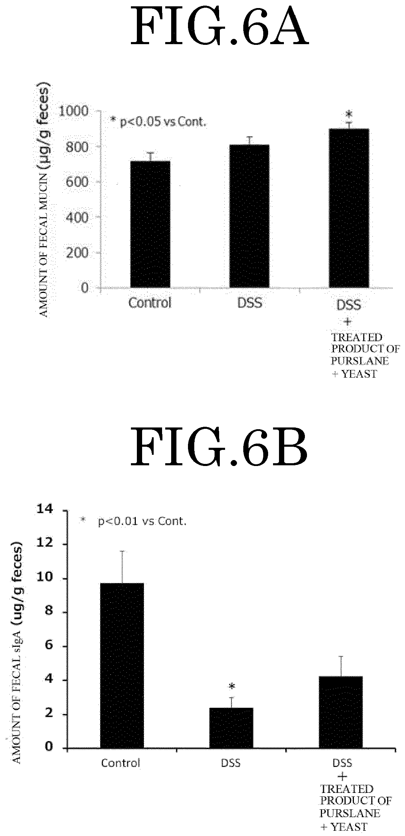

The measured results of the amount of fecal mucin 14 days after the start of the test are shown in FIG. 6(a). The amount of mucin was significantly increased in the DSS + "treated product of purslane + yeast" group, as compared to the Control group. Further, the measured results of the amount of fecal sIgA 14 days after the start of the test are shown in FIG. 6(b). A significant decrease in the amount of sIgA was observed in the DSS group, as compared to the Control group. However, in the DSS + "treated product of purslane + yeast" group, no significant decrease was observed as compared to the Control group (p-value: 1%), and the amount of sIgA was increased as compared to the DSS group.

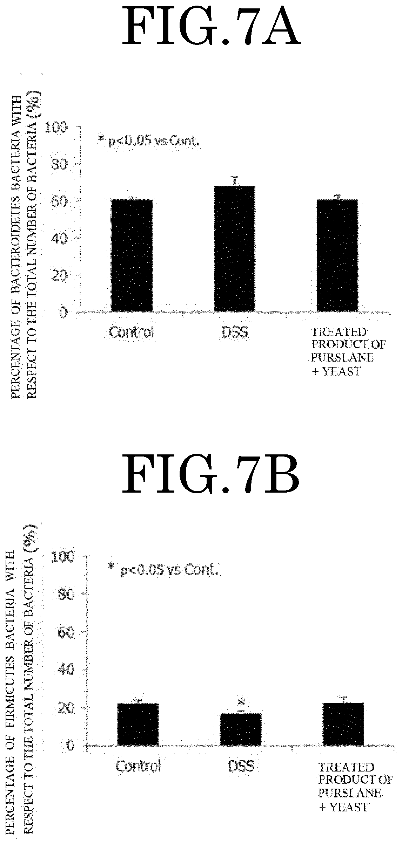

The results of the simple microbiota analysis are shown in FIG. 7. Bacteria belonging to the phylum Bacteroidetes shown in FIG. 7(a) are one of the major constituent bacteria of the intestinal microbiota, and many of the bacteria belonging to the phylum Bacteroidetes are anaerobic. There has been a report that the growth of bacteria belonging to the phylum Bacteroidetes was observed in mice with DSS-induced enteritis. Bacteria belonging to the phylum Firmicutes shown in FIG. 7(b) are a group of gram-positive bacteria including lactic acid bacteria, *Bacillus subtilis, Staphylococcus, Clostridium*, and the like, and inhabit in various places, as enteric bacteria, resident skin bacteria and pathogenic bacteria, and in fermented food.

The percentages of bacteria belonging to the phylum *Bacteroidetes* with respect to the total number of bacteria, in the feces collected 14 days after the start of the test in the respective groups, are shown in FIG. 7(a). In the DSS group, a tendency of an increase in the percentage of bacteria belonging to the phylum *Bacteroidetes* was observed, as compared to the Control group. However, in the DSS + "treated product of purslane + yeast" group, a tendency of a decrease in the percentage of bacteria belonging to the phylum *Bacteroidetes* was observed, as compared to the DSS group.

The percentages of bacteria belonging to the phylum *Firmicutes* with respect to the total number of bacteria, in the feces collected 14 days after the start of the test in the respective groups, are shown in FIG. 7(b). In the DSS group, the percentage of bacteria belonging to the phylum *Bacteroidetes* significantly decreased as compared to the Control group. However, in the DSS + "treated product of purslane + yeast" group, a tendency of an increase in the percentage of bacteria belonging to the phylum *Bacteroidetes* was observed, as compared to the DSS group.

As can be seen from the above results, the administration of the treated product of purslane + yeast prevented the decrease in the amount of mucin and the decrease in the amount of sIgA caused by the administration of DSS, and also prevented the aggravation of the intestinal microbiota. The increase in the amount of mucin suggests that the intestinal microbiota has been stabilized.

(Example 3)

An in-house monitor test was carried out as follows.

As a preliminary human monitor test, an in-house monitor test was carried out on three subjects. The purpose of this test is to examine whether or not the ingestion of the treated product of purslane + yeast increases the amount of mucin in human intestines.

The test was carried out as an open label method, and capsules containing the treated product of purslane + yeast (in an amount corresponding to 400 mg/day of the treated product of purslane + yeast, in terms of the solid content), prepared in accordance with the method of Example 1, were administered to the three in-house subjects for three weeks. Before and after the ingestion of test food, feces and blood were collected from each subject.

The evaluation was carried out on the following points.

・ Comprehensive intestinal microbiota analysis (next-generation sequencer)

The feces collected using a stool collection kit (product name: Stool Collection Kit, manufactured by Techno-Suruga Laboratory Co., Ltd.) were stored frozen. The stored feces were subjected to an entrusted analysis of intestinal microbiota (metagenomic analysis by a next-generation sequencer) provided by Cosmo Bio Co., Ltd. The principal component analysis was carried out using BaseSpace Sequence Hub, manufactured by Illumina, Inc.

Amount of fecal mucin

The measurement was carried out using a kit for measuring fecal mucin (product name: Fecal Mucin Assay Kit, manufactured by Cosmo Bio Co., Ltd.).

Amount of fecal sIgA

The feces collected using a stool collection kit (product name: Stool Collection Kit, manufactured by Techno-Suruga Laboratory Co., Ltd.) were stored frozen. The stored feces were subjected to a fecal sIgA measurement analysis provided by Cosmo Bio Co., Ltd.

・ Questionnaire (stomach conditions and defecation status)

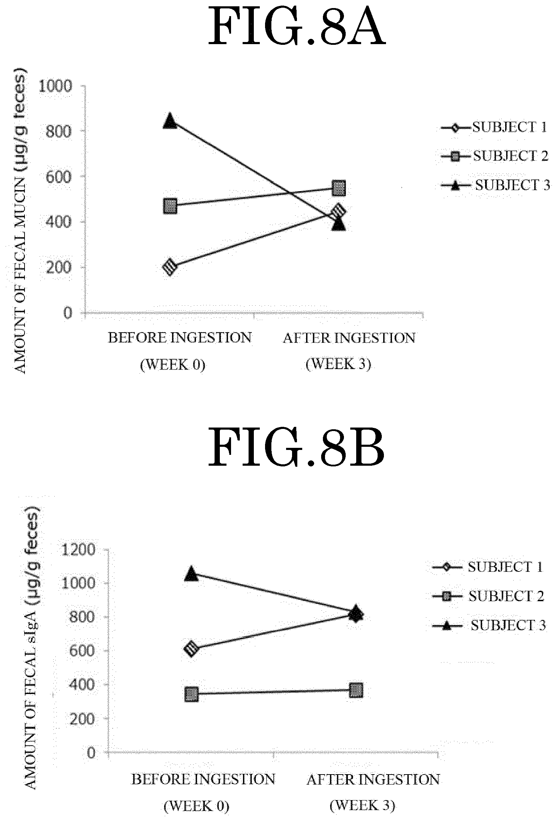

The measured results of the amount of fecal mucin and the amount of fecal sIgA are shown in FIG. 8. As a result of ingesting the capsules containing the treated product of purslane + yeast, an increase in the amount of fecal mucin was observed in two out of three subjects (FIG. 8(a)), and an increase in the amount of sIgA was also observed in two out of three subjects (FIG. 8(b)).

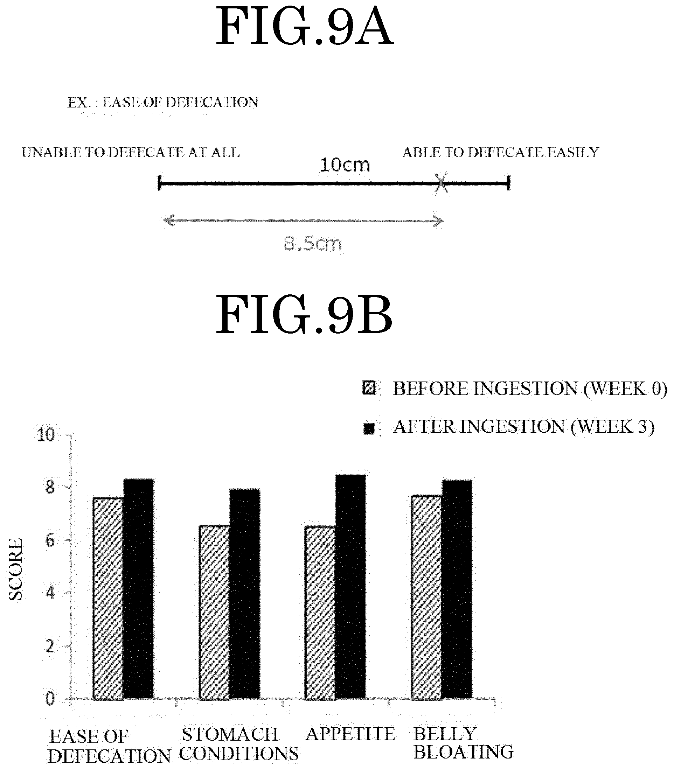

The evaluation of stomach conditions was carried out using a VAS for each item, which is a method of digitizing the results of a questionnaire (FIG. 9(a)). As shown in FIG. 9(b), a tendency of an increase in the overall scores was observed in the items regarding ease of defecation, stomach conditions, appetite and belly bloating, after the ingestion of the capsules, as compared to before the ingestion.

A questionnaire regarding the defecation status before and after the ingestion was carried out, on the following items.

・ Stool form (method using the Bristol scale)

7: Separate lumpy stool (separate hard lumps, like nuts)
6: Hard stool (sausage-shaped, but lumpy)
5: Semi-hard stool (sausage-shaped, with cracks on the surface)

4: Normal stool (like a soft sausage or a coiling snake, with smooth surface)
3: Semi-soft stool (soft, semi-solid blobs with clear-cut edges)
2: Mushy stool (fluffy pieces with ragged ages, a mushy stool)
1: Watery stool (watery, with no solid pieces. Entirely liquid)

˙ Stool color

The stool color was evaluated in five levels in a scale of "1" to "5", corresponding to "bad" to "good".

˙ Stool size

Stools were likened to columns having a diameter of 25 mm × length of 57 mm, and the number of the columns was filled in.

˙ Stool odor

1: very strong, 2: strong, 3: normal, 2: weak, 1: very weak

˙ Defecation time

The time required from the entrance to the bath room until defecation was filled in.

˙ Difficulty in defecation

1: needed to strain strongly, 2: needed to strain to some extent, 3: hardly needed to strain

˙ Feeling after defecation

1: no refreshed feeling, 2: normal, 3: felt refreshed

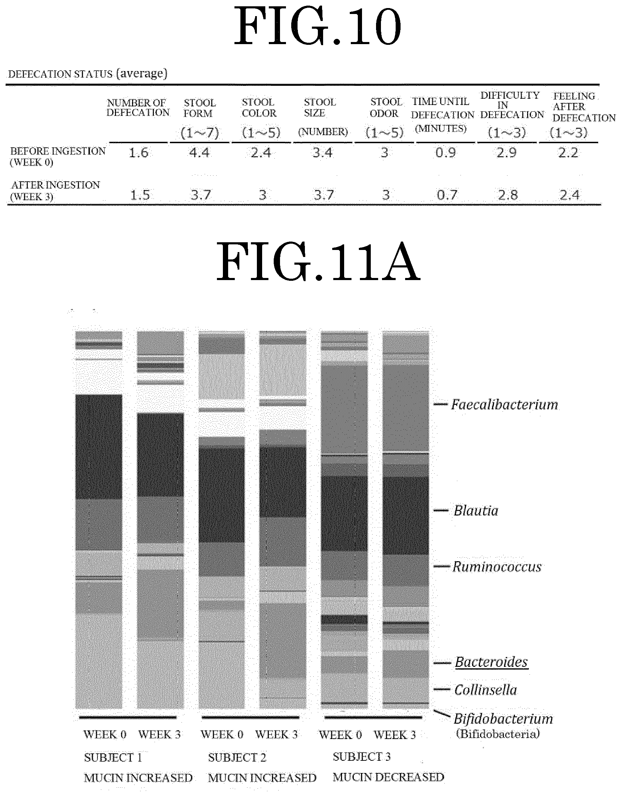

The results of the questionnaire revealed, as shown in FIG. 10, the mean value of the stool color, in particular, changed from 2.4 to 3.0, nearing to 5 which indicates a favorable condition, and the mean value of the feeling after defecation also changed from 2.2 to 2.4, nearing to 3, which indicates a refreshed feeling.

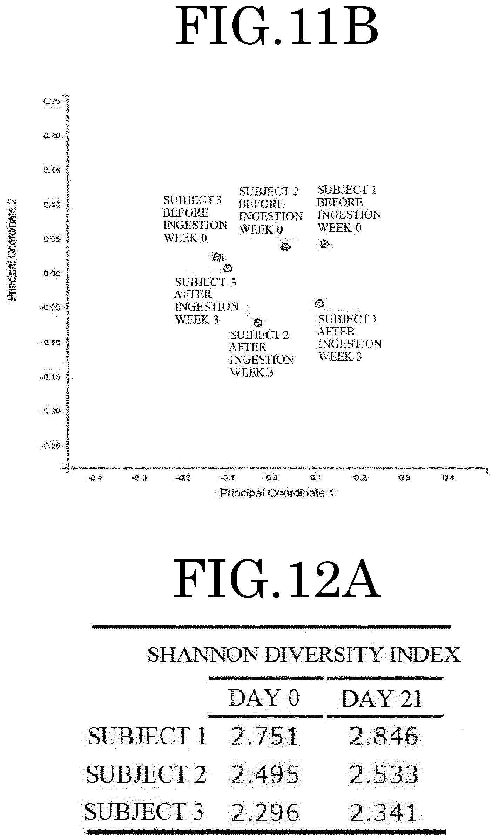

The results of the comprehensive analysis of the intestinal microbiota are shown in FIG. 11(a). Subjects 1 and 2, in whom an increase in the amount of mucin was observed in FIG. 8(a), have a similar composition of the intestinal microbiota, and showed a similar behavior in changes before and after the ingestion, differing from Subject 3. Further, an increase in the number of bacteria belonging to the genus *Bacteroides*, which provide beneficial effects on the intestinal immune system, was observed in in all the subjects. In addition, the results of the principal component analysis (PCA) of the intestinal microbiota are shown in FIG. 11(b). Since the positions of the points representing Subjects 1 and 2 in FIG. 11(b) are closer to each other, as compared to the position of Subject 3, it can be confirmed that Subjects 1 and 2 have a similar composition of the intestinal microbiota. Further, since Subjects 1 and 2 showed greater changes before and after the ingestion, as compared to Subject 3, it can be confirmed that the changes in the composition of the intestinal microbiota of Subjects 1 and 2 were greater than that of Subject 3.

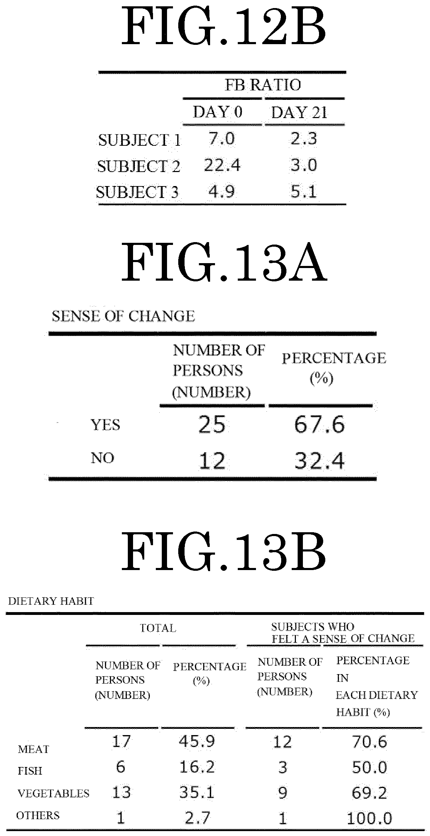

The analysis results of: the diversity of intestinal bacteria; an index indicating susceptibility to weight gain, due to the composition of the intestinal microbiota; and the trend of changes in the intestinal microbiota; are shown in FIG. 12. The Shannon index is an index for α diversity, and a larger value of the Shannon index indicates a greater diversity of intestinal bacteria. It can be seen from FIG. 12(a) that the diversity of intestinal bacteria increased after the ingestion. The Firmicutes/Bacteroidetes (FB) ratio is the ratio of the number of bacteria belonging to the phylum *Firmicutes* to the number of bacteria belonging to the phylum *Bacteroidetes,* and it is considered that a lower FB ratio indicates a lower susceptibility to weight gain. It can be seen from FIG. 12(b) that the composition of the intestinal microbiota has changed to that leading a lower susceptibility to weight gain.

As described above, the amount of fecal mucin and the amount of fecal sIgA increased in two out of three subjects, as a result of the in-house monitor test. Further, the sense of change was confirmed in the improvement in the stomach conditions, as well as in the improvement in the stool conditions. In addition, an increase in the number of bacteria belonging to the phylum *Bacteroidetes*, which are deeply related to the intestinal immune system, and an increase in the diversity of intestinal bacteria were observed.

(Example 4)

A larger-scale in-house monitor test was carried out as follows.

The in-house monitor test was carried out on 37 subjects who volunteered to ingest the treated product of purslane + yeast. The purpose of this test is to examine the sense of change obtained by ingesting the treated product of purslane + yeast.

As the test food, the same capsules as those used in Example 3, containing the treated product of purslane + yeast (in an amount corresponding to 400 mg/ day of the treated product of purslane + yeast, in terms of the solid content) were used. Each subject ingested the capsules for 14 days. The subjects were allowed to ingest the capsules at any time in a day, but required to ingest at the same time, every day.

The evaluation was carried out on the following items. The subjects were asked to fill out two types of questionnaires (after the ingestion, only).

. Questionnaire 1: regarding the presence or absence of a sense of change, time of ingestion, dietary habit, and the like
. Questionnaire 2: regarding improvements in physical conditions after the ingestion (stomach and intestines, eyes, nose, ears, mouth, skin, legs, joints, brain, immune system, and the like)

The administration schedule will be described below. The ingestion started on December 12, 2016, and completed on December 26, 2016. The questionnaires were filled on the day of completion of the ingestion. The subjects were allowed to live normal lives without any limitations on meals, alcohols, and the like. Those on medications were also allowed to participate in the test.

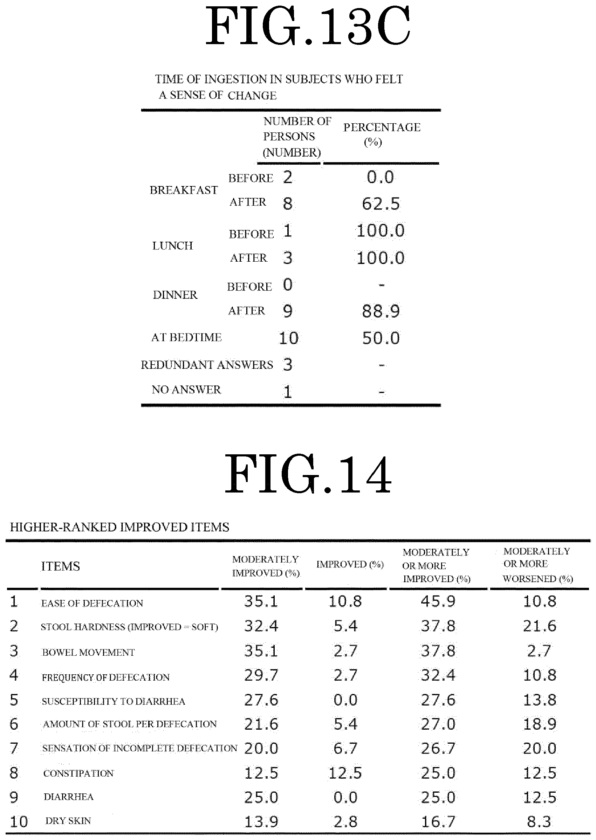

The results of the questionnaire 1 are shown in FIG. 13. FIG. 13(a) shows that 67.6% of the subjects answered that they felt a sense of change. FIG. 13(b) shows that good reactivity was observed in subjects on a diet mainly based on meat and vegetables. FIG. 13(c) shows that the ingestion during the period between lunch and dinner resulted in a better reactivity.

The results of the questionnaire 2 are shown in FIG. 14. It can be seen from FIG. 14 that 45.9% of the subjects answered that they felt "moderately or more improved" regarding the "ease of defecation", 37.8% of the subjects felt "moderately or more improved" regarding the "stool hardness", and 37.8% of the subjects felt "moderately or more improved" regarding the "bowel movement". Other improved items obtained as free comments include an improved concentration, a reduced sleepiness during the day, enhanced appetite, reduced coldness of hands and feet, and weight loss. The above results proved that many of the subjects felt a sense of change on the items related to the intestinal environment. Regarding the items other than those related to bowel conditions, many subjects felt an improvement in "dry skin".

As described above, the sense of change was confirmed in about 68% of the subjects, by ingesting the treated product of purslane + yeast for 14 days. Specifically, many of the subjects felt a sense of change regarding the items related to the intestinal environment, such as an improvement in defecation. Further, the effect of improving dry skin and the like has also been suggested.

(Example 5)

Out of 80 subjects, 60 subjects in whom the amount of fecal mucin was low were selected, and the test was carried out as follows.

The amount of fecal mucin was measured in 80 subjects 14 days before the start of ingestion, and 60 subjects (38 male subjects and 22 female subjects, average age $21.6 \pm 2.4$ years old) in whom the amount of fecal mucin was low were selected. Thereafter, the subjects were divided into: a placebo group consisting of 20 subjects; a low dose group consisting of 20 subjects, who ingested capsules containing 200 mg of the treated product of purslane + yeast prepared in Example 1, in terms of the solid content (200 mg/day of the treated product of purslane + yeast, in terms of the solid content); and a high dose Group consisting of 20 subjects, who ingested capsules containing 400 mg of the treated product of purslane + yeast prepared in Example 1, in terms of the solid content (400 mg/day of the treated product of purslane + yeast, in terms of the solid content); to carry out the test. The test food was ingested for a period of 28 days. The collection of stools and the filling out of the questionnaire were carried out on Day 0, Day 14, and Day 28. The measurement of the amount of fecal mucin was carried out in the same manner as in Example 3.

In the questionnaire, physical discomfort, psychological discomfort, anxiety and satisfaction were evaluated, based on the patient assessment of constipation quality of life (PAC-QOL).

Questionnaire items are as follows. Each item was evaluated in five levels in a scale of "0" to "4", corresponding to "none" to "extremely".

Physical discomfort

1. Have you felt bloated to the point of bursting?
2. Have you felt heavy because of your constipation?
3. Have you felt any physical discomfort?
4. Have you felt the need to have a bowel movement but not been able to? Psychosocial discomfort
5. Have you been embarrassed to be with other people?
6. Have you been eating less and less because of not being able to have bowel movements?
7. Have you had to be careful about what you eat?
8. Have you had a decreased appetite?
9. Have you been worried about not being able to choose what you eat (for example, at a friend's house)?

10. Have you been embarrassed about staying in the bathroom for so long when you were away from home?
11. Have you been embarrassed about having to go to the bathroom so often when you were away from home?
12. Have you been worried about having to change your daily routine when you were traveling or being away from home?

Worries / concerns (anxiety)
13. Have you felt irritable because of your constipation?
14. Have you been upset by your constipation?
15. Have you felt obsessed by your constipation?
16. Have you felt stressed by your constipation?
17. Have you felt less self-confident because of your constipation?
18. Have you felt in control of your situation
19. Have you been worried about not knowing when you are going to be able to have a bowel movement?
20. Have you been worried about not being able to have a bowel movement?
21. Have you been more and more bothered by not being able to have a bowel movement?
22. Have you been worried that your condition will get worse?
23. Have you felt that your body was not working properly? Satisfaction
24. Have you had fewer bowel movements than you would like?
25. Have you been satisfied with how often you have a bowel movement?
26. Have you been satisfied with the regularity of your bowel movements?
27. Have you been satisfied with your intestinal function?
28. Have you been satisfied with your treatment?

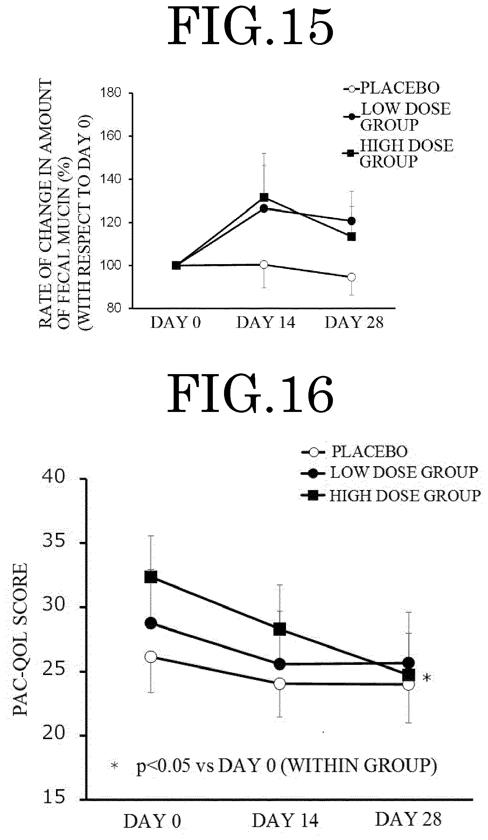

The results of the measurement of the amount of fecal mucin are shown in FIG. 15. An increase in the amount of fecal mucin was observed in the low dose group and the high dose group, as compared to the placebo group.

The results of the PAC-QOL (the total score of the physical discomfort, psychological discomfort, anxiety and satisfaction) are shown in FIG. 16. A lower value of the PAC-QOL score indicates a more improved QOL which has been impaired by constipation. In the high dose group, a significant improvement in QOL was observed as compared to the placebo group.

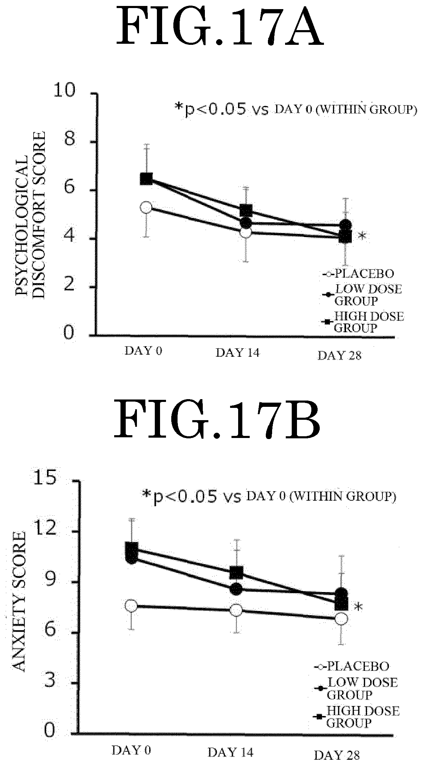

Of the results of the PAC-QOL, the results of the psychological discomfort score are shown in FIG. 17(a), and the results of the anxiety score are shown in FIG. 17(b). In the high dose group, significant improvements in both the psychological discomfort score and the anxiety score were observed, as compared to the placebo group. This has proven that the treated product of purslane according to the present Example improves the QOL which has been impaired by constipation.

As described above, it has been shown that the treated product of purslane (the treated product of purslane + yeast, or the pectinase-treated product of purslane + yeast) according to the present Example has excellent effects of increasing mucin gene expression, increasing the amount of IgA and improving the intestinal microbiota, and thus is useful as a supplement, a pharmaceutical, an agent for protecting intestinal mucosa and an intestinal regulator.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2017-108131, filed on May 31, 2017, the entire disclosure of which is incorporated by reference herein.

## Claims

1. A treated product of purslane obtained by treating purslane with hot water to obtain a hot water extract, and adding yeast to the hot water extract and culturing a resultant.

2. The treated product of purslane according to claim 1, wherein at least one of pectinase or cellulase is further added to the hot water extract.

3. A method of producing a treated product of purslane, the method comprising:

   a hot water treatment step of treating purslane with hot water; and
   a culturing step of adding yeast, and culturing a resultant.

4. The method of producing a treated product of purslane according to claim 3, wherein at least one of pectinase or cellulase is further added, in the culturing step.

5. A supplement comprising, as an effective ingredient, a treated product of purslane obtained by treating purslane with hot water to obtain a hot water extract, and adding yeast to the hot water extract and culturing a resultant.

6. The supplement according to claim 5, wherein at least one of pectinase or cellulase is further added to the hot water extract.

7. A pharmaceutical comprising, as an effective ingredient, a treated product of purslane obtained by treating purslane with hot water to obtain a hot water extract, and adding yeast to the hot water extract and culturing a resultant.

8. The pharmaceutical according to claim 7, wherein at least one of pectinase or cellulase is further added to the hot water extract.

9. An agent for protecting intestinal mucosa, comprising, as an effective ingredient, a treated product of purslane obtained by treating purslane with hot water to obtain a hot water extract, and adding yeast to the hot water extract and culturing a resultant.

10. The agent for protecting intestinal mucosa according to claim 9, wherein at least one of pectinase or cellulase is further added to the hot water extract.

11. An intestinal regulator comprising, as an effective ingredient, a treated product of purslane obtained by treating purslane with hot water to obtain a hot water extract, and adding yeast to the hot water extract and culturing a resultant.

12. The intestinal regulator according to claim 11, wherein at least one of pectinase or cellulase is further added to the hot water extract.

# FIG.1

| NAMES OF MATERIALS | MUC2 GENE EXPRESSION (%) (RATIO COMPARED TO NON-TREATED SAMPLES) |
|---|---|
| TREATED PRODUCT OF PURSLANE + YEAST | 272 |
| HOT WATER EXTRACT OF PURSLANE | 157 |
| TREATED PRODUCT OF MELON + YEAST | 113 |
| HOT WATER EXTRACT OF MELON | 94 |

# FIG.2A

CONT. OF TREATED PRODUCT OF PURSLANE + YEAST (μg/mL)

# FIG.2B

# FIG.3

# FIG.4

MUC2 GENE EXPRESSION LEVEL (% OF CONT.)

* p<0.01 vs Control

AMOUNT OF YEAST ADDED
(WITH RESPECT TO AMOUNT OF RAW MATERIAL)

# FIG.5

MUC2 GENE EXPRESSION LEVEL (% OF CONT.)

* p<0.01 vs Cont.

CULTURE TIME

# FIG.6A

# FIG.6B

# FIG.7A

# FIG.7B

# FIG.8A

# FIG.8B

# FIG.9A

EX. : EASE OF DEFECATION

UNABLE TO DEFECATE AT ALL                                      ABLE TO DEFECATE EASILY

10cm

8.5cm

# FIG.9B

☒ BEFORE INGESTION (WEEK 0)
■ AFTER INGESTION (WEEK 3)

SCORE

EASE OF DEFECATION   STOMACH CONDITIONS   APPETITE   BELLY BLOATING

# FIG.10

DEFECATION STATUS (average)

| | NUMBER OF DEFECATION | STOOL FORM (1~7) | STOOL COLOR (1~5) | STOOL SIZE (NUMBER) | STOOL ODOR (1~5) | TIME UNTIL DEFECATION (MINUTES) | DIFFICULTY IN DEFECATION (1~3) | FEELING AFTER DEFECATION (1~3) |
|---|---|---|---|---|---|---|---|---|
| BEFORE INGESTION (WEEK 0) | 1.6 | 4.4 | 2.4 | 3.4 | 3 | 0.9 | 2.9 | 2.2 |
| AFTER INGESTION (WEEK 3) | 1.5 | 3.7 | 3 | 3.7 | 3 | 0.7 | 2.8 | 2.4 |

# FIG.11A

— Faecalibacterium

— Blautia

— Ruminococcus

— Bacteroides

— Collinsella

— Bifidobacterium (Bifidobacteria)

WEEK 0   WEEK 3     WEEK 0   WEEK 3     WEEK 0   WEEK 3

SUBJECT 1          SUBJECT 2          SUBJECT 3

MUCIN INCREASED   MUCIN INCREASED   MUCIN DECREASED

# FIG.11B

# FIG.12A

| | SHANNON DIVERSITY INDEX | |
|---|---|---|
| | DAY 0 | DAY 21 |
| SUBJECT 1 | 2.751 | 2.846 |
| SUBJECT 2 | 2.495 | 2.533 |
| SUBJECT 3 | 2.296 | 2.341 |

# FIG.12B

|  | FB RATIO | |
| --- | --- | --- |
|  | DAY 0 | DAY 21 |
| SUBJECT 1 | 7.0 | 2.3 |
| SUBJECT 2 | 22.4 | 3.0 |
| SUBJECT 3 | 4.9 | 5.1 |

# FIG.13A

SENSE OF CHANGE

|  | NUMBER OF PERSONS (NUMBER) | PERCENTAGE (%) |
| --- | --- | --- |
| YES | 25 | 67.6 |
| NO | 12 | 32.4 |

# FIG.13B

DIETARY HABIT

|  | TOTAL | | SUBJECTS WHO FELT A SENSE OF CHANGE | |
| --- | --- | --- | --- | --- |
|  | NUMBER OF PERSONS (NUMBER) | PERCENTAGE (%) | NUMBER OF PERSONS (NUMBER) | PERCENTAGE IN EACH DIETARY HABIT (%) |
| MEAT | 17 | 45.9 | 12 | 70.6 |
| FISH | 6 | 16.2 | 3 | 50.0 |
| VEGETABLES | 13 | 35.1 | 9 | 69.2 |
| OTHERS | 1 | 2.7 | 1 | 100.0 |

EP 3 632 455 A1

# FIG.13C

TIME OF INGESTION IN SUBJECTS WHO FELT A SENSE OF CHANGE

| | | NUMBER OF PERSONS (NUMBER) | PERCENTAGE (%) |
|---|---|---|---|
| BREAKFAST | BEFORE | 2 | 0.0 |
| | AFTER | 8 | 62.5 |
| LUNCH | BEFORE | 1 | 100.0 |
| | AFTER | 3 | 100.0 |
| DINNER | BEFORE | 0 | - |
| | AFTER | 9 | 88.9 |
| AT BEDTIME | | 10 | 50.0 |
| REDUNDANT ANSWERS | | 3 | - |
| NO ANSWER | | 1 | - |

# FIG.14

HIGHER-RANKED IMPROVED ITEMS

| | ITEMS | MODERATELY IMPROVED (%) | IMPROVED (%) | MODERATELY OR MORE IMPROVED (%) | MODERATELY OR MORE WORSENED (%) |
|---|---|---|---|---|---|
| 1 | EASE OF DEFECATION | 35.1 | 10.8 | 45.9 | 10.8 |
| 2 | STOOL HARDNESS (IMPROVED = SOFT) | 32.4 | 5.4 | 37.8 | 21.6 |
| 3 | BOWEL MOVEMENT | 35.1 | 2.7 | 37.8 | 2.7 |
| 4 | FREQUENCY OF DEFECATION | 29.7 | 2.7 | 32.4 | 10.8 |
| 5 | SUSCEPTIBILITY TO DIARRHEA | 27.6 | 0.0 | 27.6 | 13.8 |
| 6 | AMOUNT OF STOOL PER DEFECATION | 21.6 | 5.4 | 27.0 | 18.9 |
| 7 | SENSATION OF INCOMPLETE DEFECATION | 20.0 | 6.7 | 26.7 | 20.0 |
| 8 | CONSTIPATION | 12.5 | 12.5 | 25.0 | 12.5 |
| 9 | DIARRHEA | 25.0 | 0.0 | 25.0 | 12.5 |
| 10 | DRY SKIN | 13.9 | 2.8 | 16.7 | 8.3 |

29

# FIG.15

# FIG.16

# FIG.17A

# FIG.17B

**INTERNATIONAL SEARCH REPORT**

| International application No. |
| --- |
| PCT/JP2018/020866 |

A. CLASSIFICATION OF SUBJECT MATTER
Int.Cl.   A61K36/185(2006.01)i,   A23L33/105(2016.01)i,   A61K36/06(2006.01)i,
          A61P1/04(2006.01)i, A61P1/12(2006.01)i, A61P43/00(2006.01)i
According to International Patent Classification (IPC) or to both national classification and IPC

B.   FIELDS SEARCHED

Minimum documentation searched (classification system followed by classification symbols)
Int.Cl. A61K36/185, A23L33/105, A61K36/06, A61P1/04, A61P1/12, A61P43/00

| Documentation searched other than minimum documentation to the extent that such documents are included in the fields searched | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII)
CAplus/MEDLINE/EMBASE/BIOSIS (STN)

C.   DOCUMENTS CONSIDERED TO BE RELEVANT

| Category* | Citation of document, with indication, where appropriate, of the relevant passages | Relevant to claim No. |
| --- | --- | --- |
| X | CN 103045453 A (XUZHOU LYUZHIYE BIOLOGICAL FOOD CO., LTD.) 17 April 2013, paragraph [0017] (Family: none) | 1, 3 |
| X | KR 10-1457960 B1 (LG HOUSEHOLD & HEALTH CARE LTD.) 04 November 2014, paragraphs [0023], [0029], [0044], [0059]-[0064] (Family: none) | 1, 3 |
| Y | | 2, 4 |

☒   Further documents are listed in the continuation of Box C.       ☐   See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 August 2018 (24.08.2018) | 04 September 2018 (04.09.2018) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**EP 3 632 455 A1**

**INTERNATIONAL SEARCH REPORT**

| International application No. |
| --- |
| PCT/JP2018/020866 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2013-172711 A (KANAZAWA INSTITUTE OF TECHNOLOGY) 05 September 2013, paragraph [0008] (Family: none) | 1-12 |
| Y | JP 2008-247779 A (NIPPON TABLET KK) 16 October 2008, paragraph [0044] (Family: none) | 1-8 |
| Y | CN 101181317 A (SECOND MILITARY MEDICAL UNIVERSITY OF PLA) 21 May 2008, example 2 (Family: none) | 1-8 |
| Y | KR 10-2011-0060238 A (AMOREPACIFIC CORPORATION) 08 June 2011, test example 2 (Family: none) | 1-8 |
| Y | TAO, Zhang et al., "Effect of portulaca oleracea polysaccharide on treating ulcerative colitis induced by TNBs in rats", Zhongguo Zhongxiyi Jiehe Xiaohua Zazhi, 2009, 17(2) 96-99, (abstract) CAplus [online], US: American Chemical Society [search date 24 August 2018] Retrieved from: SIN, Accession No. 152:397084, entire text | 1-12 |
| Y | GONG, Fayong et al., "Hypoglycemic effects of crude polysaccharide from purslane", Int. J. Mol. Sci, 2009, 10, 880-888, doi: 10.3390/ijms10030880, page 884, column 3.3 | 1-12 |
| Y | 福田伊津子 他, パン酵母 β-グルカンの整腸作用について, 日本醸造協会誌, 2009, 104(12), 939-943, page 941, column 3.2 to page 942, column 4, (FUKUDA, Itsuko et al., "Amelioration of Intestinal Function by Baker's Yeast-Derived β-Glucan", Journal of the Brewing Society of Japan) | 1-12 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017108131 A

**Non-patent literature cited in the description**

- **ZHANG TAO ; WANG XIAO-PING ; CHCN JIAN-YONG ; PAN FENG.** Effect of portulaca oleracea polysaccharide on treating ulcerative colitis induced by TNBs in rats. *Chinese Journal of Integrated Traditional and Western Medicine on Digestion,* 2009, vol. 17 (2), 96-99

- *Isokinetics and Exercise Science,* 2011, vol. 19, 199-206
- **TAIRA.** *J Clin Biochem Nutr,* 2015, vol. 56, 149-54